# EUROPEAN PATENT APPLICATION

(11) **EP 4 609 839 A1**
(43) Date of publication of application: **03.09.2025**
(21) Application number: 24160184.8
(22) Date of filing: 28.02.2024
(51) Int. Cl.: A61F 9/007, A61B 90/00

(54) **SURGICAL ROBOTIC SYSTEM AND CONTROL OF SURGICAL ROBOTIC SYSTEM**

(71) Applicant: Carl Zeiss Meditec AG, 07745 Jena (DE)
(72) Inventor: HILLENBRAND, Matthias, 73447 Oberkochen (DE); BRIEL, Marius, 76344 Eggenstein-Leopoldshafen (DE); HAIDE, Ludwig, 76344 Eggenstein-Leopoldshafen (DE); VOIGT, Christian, 73447 Oberkochen (DE); BEELEN, Maarten, 5612 AP Eindhoven (NL); NAUS, Gerrit Jacobus Lambertus, 5612 AP Eindhoven (NL); DA COL, Tommaso, 5612 AP Eindhoven (NL)
(74) Representative: DeltaPatents B.V.

(57) **Abstract**

A surgical robotic system is provided for use in an intraocular procedure, comprising a surgical arm comprising a movable arm part, the movable arm part comprising an end effector for holding a surgical instrument, an OCT probe configured to attach to or integrate into an intraocular part of the surgical instrument, and an actuator subsystem configured to actuate the movable arm part. During the intraocular procedure, a presence of a feature of interest may be monitored by repeatedly, at respective positions of the surgical instrument, acquiring sensor data via the OCT probe, analysing the reflectivity depth profile to determine an attribute of the feature of interest in the reflectivity depth profile, and if the attribute meets a predetermined attribute criterion, effecting a feedback action. Thereby, a human operator may be alleviated from having to mentally determine the attribute of the feature of interest, and feedback actions may contribute to carrying out (semi-)autonomous operations.

## Description

### TECHNICAL FIELD

The invention relates to a surgical robotic system for use in an intraocular procedure. The invention further relates to a computer-implemented method for controlling a surgical robotic system during an intraocular procedure, and to a computer program product comprising instructions to perform the method.

### BACKGROUND

Intraocular procedures increasingly involve the use of surgical robotic systems. Rather than operating entirely autonomously, such surgical robotic systems are expected for the foreseeable future to remain at least in part under the control of a human operator. For example, the human operator may directly or indirectly control the movement of a surgical instrument mounted to a surgical arm of the surgical robotic system. Nevertheless, it is expected that future surgical robotic systems may be more autonomous and require less or even no involvement of a human operator.

A surgical robotic system designed for intraocular procedures may be provided with a surgical arm which comprises a movable arm part, with the movable arm part comprising an end effector for holding a surgical instrument. Accordingly, the surgical instrument may be positioned by the surgical arm. An actuator subsystem may be provided for actuating the movable arm part to effect a movement of the surgical instrument. The movement of the surgical instrument may be in a lateral direction, for example relative to a retinal surface, as well as in a longitudinal direction, e.g., along the longitudinal axis of the surgical instrument. Such longitudinal movement may allow the surgical instrument to be moved towards and away from a surgical target within an interior of the eye. Accordingly, the surgical instrument may be used to modify (biological) tissue near the surgical target, to deliver an agent to the surgical target, etc. Examples of surgical instruments include, but are not limited to, forceps, mechanical cutters, coagulation cutters, scissors, injection needles, sealing devices, etc.

Surgical robotic systems of the above type are known per se. For example, WO 2016/030336 A1 describes a surgical robotic system for use in a surgical procedure, comprising a surgical arm comprising a movable arm part, the movable arm part comprising an instrument connector for mounting of a surgical instrument, the surgical instrument having a longitudinal axis, the movable arm part having at least one degree-of-freedom to enable longitudinal movement of the surgical instrument along the longitudinal axis of the surgical instrument towards a surgical target. The surgical robotic system is said to further comprise a human machine interface for receiving positioning commands from a human operator for controlling the longitudinal movement of the surgical instrument, an actuator configured for actuating the movable arm part to effect the longitudinal movement of the surgical instrument, and a processor configured for controlling the actuator in accordance with the positioning commands.

### SUMMARY

During an intraocular procedure, attributes of features nearby the surgical instrument may be of particular interest. For example, in a membrane peeling procedure, it may be of interest whether the epiretinal membrane is present nearby the surgical instrument and/or whether the epiretinal membrane has a certain thickness.

It would be advantageous for a surgical robotic system to be able to assist in determining the attribute of such features of interest.

In a first aspect of the invention, a surgical robotic system is provided for use in a surgical procedure, such as an intraocular procedure. The surgical robotic system comprises:
- a surgical arm comprising a movable arm part, the movable arm part comprising an end effector for holding a surgical instrument;
- an optical coherence tomography, OCT, probe, wherein the OCT probe is configured to attach to or integrate into an insertable part, such as an intraocular part, of the surgical instrument for being inserted into an interior of a patient, for example into the eye;
- an actuator subsystem configured to actuate the movable arm part;
- a processor subsystem configured to control the actuator subsystem to control a position of the surgical instrument during the surgical procedure, wherein the processor subsystem is further configured to monitor for a feature of interest by repeatedly, at respective positions of the surgical instrument:
- acquire sensor data via the OCT probe, wherein the sensor data is indicative of a reflectivity depth profile originating from structures within a beam of the OCT probe;
- analyse the reflectivity depth profile to determine an attribute of the feature of interest in the reflectivity depth profile; and
- if the attribute meets a predetermined attribute criterion, effect a feedback action.

In a further aspect of the invention, a computer-implemented method is provided for controlling a surgical robotic system during a surgical procedure, such as an intraocular procedure. The surgical robotic system comprises:
- a surgical arm comprising a movable arm part, the movable arm part comprising an end effector for holding a surgical instrument;
- an optical coherence tomography, OCT, probe, wherein the OCT probe is configured to attach to or integrate into an insertable part, such as an intraocular part, of the surgical instrument for being inserted into an interior of a patient, for example into the eye;
- an actuator subsystem configured to actuate the movable arm part;

The method comprises:
- controlling the actuator subsystem to control a position of the surgical instrument during the surgical procedure; and
- simultaneously or alternatingly in time with said controlling, monitoring for a feature of interest by repeatedly, at respective positions of the surgical instrument:
- acquiring sensor data via the OCT probe, wherein the sensor data is indicative of a reflectivity depth profile originating from structures within a beam of the OCT probe;
- analysing the reflectivity depth profile to determine an attribute of the feature of interest in the reflectivity depth profile; and
- if the attribute meets a predetermined attribute criterion, effecting a feedback action.

In a further aspect of the invention, a transitory or non-transitory computer-readable medium is provided comprising data representing a computer program, the computer program comprising instructions for causing a processor system to perform the above-identified computer-implemented method.

The above aspects of the invention provide a surgical robotic system which comprises a surgical arm. The surgical arm may comprise a movable arm part, and the movable arm part may comprise an end effector for holding a surgical instrument. For example, the end effector may comprise an instrument connector for mounting the surgical instrument, e.g., in a removable manner. The surgical instrument may typically have a longitudinal axis which may typically pass through a tip of the surgical instrument. The movable arm part may have at least one Degrees-of-Freedom (DoF) to move the tip of the surgical instrument, for example laterally in relation to an anatomical surface, such as a retinal surface or the surface of another structure within or on the eye. The movable arm part may typically have three or more DoFs, for example to enable longitudinal movement of the surgical instrument along the longitudinal axis, for example to advance towards and/or retract away from a surgical target, and to enable lateral movement of the tip of the surgical instrument along a plane perpendicular to the longitudinal axis. It is noted that surgical arms having the functionality described in this paragraph are known per se from the field of medical robotics, and also known as instrument manipulators, robotic arms, surgical robot slave devices, etc.

An actuator subsystem may be provided for actuating the movable arm part to effect the lateral movement, and typically also longitudinal movement, of the tip surgical instrument. Another term for actuator subsystem is driving mechanism.

A processor subsystem may be provided for controlling the actuator subsystem to control a position of the surgical instrument during the surgical procedure, for example relative to an intraocular surface during an intraocular procedure. In some embodiments, the processor subsystem may be configured to receive positioning instructions from a human operator, for example via a user input interface, and control the actuator subsystem in accordance with the positioning instructions. Such type of control may also be referred to as master-slave control. In some embodiments, the processor subsystem may be configured to enable a human operator to exclusively control the position of the surgical instrument. In other embodiments, the processor subsystem may be configured to control the position of the surgical instrument semi-autonomously or even fully autonomously, for at least part of the surgical procedure. In such embodiments, there may be a possibility for the human operator to override an autonomous operation.

The processor subsystem may be further configured to, during the surgical procedure, monitor for a feature of interest. For example, in an intraocular procedure, the feature of interest may be an intraocular feature, such as a feature on or below a retinal surface, or a feature in the vitreous cavity, such as a vitreous floater, etc. In general, the feature of interest may be an anatomical feature, e.g., an anatomical structure, tissue type, organ, etc., or in some cases a foreign object. To monitor for the feature of interest, the processor subsystem may make use of the sensor data acquired by an optical coherence tomography (OCT) probe which may be attached to or integrated into an insertable part of the surgical instrument. Here, the term `insertable part' may refer to a part of the surgical instrument which is configured to be inserted into an interior of the patient. For example, in case of an intraocular procedure, the part may be intended to be inserted into the eye. The OCT probe may for example be, or comprise, an optical fibre which is connected to an OCT sensor, with the OCT sensor being located more proximally, e.g., outside of the eye. The OCT probe may be forward facing, in that it may face along the longitudinal axis forward of the tip of the surgical instrument. In some embodiments, the OCT probe may additionally, or alternatively, be sideward facing. For example, the OCT probe may be a multi-directional probe with axial and side-viewing capability to be able to determine the attribute of a feature of interest sideward to the tip of the surgical instrument.

Using the OCT probe, the processor subsystem may obtain sensor data which is indicative of a reflectivity depth profile originating from structures within the OCT probe's radiating beam. By analysing the sensor data, for example using template matching or a machine-learning based technique, the attribute of the feature of interest in the reflectivity depth profile may be determined. The attribute of the feature may thus be determined by applying a feature detection technique to the sensor data. The attribute may for example be a presence status of the feature of interest and/or one or more characteristics of the feature of interest. Determining the attribute may comprise identifying a value of the attribute, e.g., 'present' or 'absent' for a presence status or '*n* mm' for a size dimension, etc. As such, determining the attribute of the feature of interest may refer to identifying the attribute's value.

Said steps of acquiring the sensor data, analysing the sensor data, and determining the attribute of the feature of interest may be repeated for different positions of the tip of the surgical instrument, thereby establishing the monitoring of the attribute of the feature of interest. The processor subsystem may be further configured to effect a feedback action depending on the attribute of the feature of interest meeting a predetermined criterion. For example, if the attribute is a presence status of the feature of interest and if the predetermined attribute criterion requires the presence status to be equal to 'present', the processor subsystem may effect the feedback action if the attribute is determined to be present. In another example, if the predetermined attribute criterion requires the presence status to be equal to 'absent', the processor subsystem may effect the feedback action if the attribute is determined to be absent. In another example, if the attribute is a thickness of the feature of interest and if the predetermined attribute criterion requires the thickness of the feature of interest to be equal or larger than 10 micrometres, the processor subsystem may effect the feedback action if the thickness is indeed determined to be equal or larger than 10 micrometres.

In general, the predetermined attribute criterion may define or may be indicative of a reference value and an operator. For example, if the attribute is a presence status of the feature of interest, e.g., the aforementioned 'present' or 'absent', the predetermined attribute criterion may require the attribute value to be equal to one of the possible values of the attribute, e.g., 'present'. The attribute may also be a characteristic of the feature of interest, in which case the predetermined attribute criterion may define a numerical or other quantifiable reference for the attribute value and a criterion, such as `equal to', `larger than', smaller than', etc. The characteristic may for example be a distance, a size, a dimension (e.g., a length, width, height, etc), an orientation, etc., of the feature of interest. In some examples, the processor subsystem may monitor for multiple attributes of the feature of interest simultaneously, for example by monitoring for a presence status and for one or more characteristics of the feature of interest, or for multiple characteristics of the feature of interest.

The above measures may have the effect that during a surgical procedure, such as an intraocular procedure, the surgical robotic system is enabled to monitor for the attribute of a feature of interest and to take feedback actions depending on its attribute value. This may be advantageous since in many intraoperative procedures, there may be features which are of relevance to the procedure and previously required a human operator, such as a surgeon, to monitor the attribute of such features. For example, in an epiretinal membrane peeling procedure, the surgeon may be occupied with determining whether the epiretinal membrane is present beneath the tip of the surgical instrument or not. This may require a significant amount of mental effort from the surgeon, which may in turn distract from controlling the surgical instrument and from the procedural steps which are to be taken. By the surgical robotic system automatically monitoring for the attribute of the feature of interest and taking feedback actions accordingly, the human operator may be alleviated, at least to a degree, from this mental task. Also, in case of a semi-autonomous or autonomous operation, feedback actions may contribute to the carrying out of the semi-autonomous or autonomous operation. For example, the surgical instrument may be differently controlled based on the attribute of the feature of interest meeting or not meeting the predetermined attribute criterion.

It is note that the feature of interest may be a specific feature. For example, the feature of interest may be selected by the user, or may be selected automatically by the processor subsystem, e.g., based on a type of surgical procedure or procedural step. It will be appreciated that in some embodiments, the processor subsystem may monitor for the attribute of a plurality of features of interest. In other words, the processor subsystem may try to detect a plurality of features of interest in the sensor data and in response to a detection take a respective feedback action.

Advantageously, the surgical robotic system may be used for various surgical procedures. For example, the surgical robotic system may be used for intraocular procedures such as the peeling of retinal membranes, for phacoemulsification, cortex removal and/or polishing during cataract surgery, for stent placement during glaucoma surgery, etc. Another example or use case may be the use of the surgical robotic system to locate places where Schlemm's canal is open during a microinvasive glaucoma surgery (MIGS). The feature of interest may thus be Schlemm's canal and the predetermined attribute criterion may be defined to require Schlemm's canal to be open. Yet another example or use case may be the use of the surgical robotic system to detect the presence of vessels during retinal photocoagulation, for example to prevent the delivery of laser power to such vessels. Yet another example or use case may be the detection of tumorous tissue or absence of critical structures (e.g., nerves, vessel) during a trans-oral laser microsurgery.

The following optional aspects are described with reference to the surgical robotic system but may equally apply to the computer-implemented method for controlling the surgical robotic system and to the corresponding computer program.

Optionally, the processor subsystem is configured to effect the feedback action by:
- generating and outputting a sensory perceptible feedback signal, such as an audible signal, a haptic signal, and/or a visual signal;
- adjusting the control of the actuator subsystem, for example to slow down or stop a movement of the surgical instrument; and/or
- control the surgical instrument or a further surgical instrument held by the surgical robotic system.

The feedback action may serve one or more of the abovementioned purposes. For example, the feedback action may serve to alert the human operator to the presence and/or absence of the feature of interest via a sensory perceptible feedback signal. In other examples, the feedback action may be an internal action, e.g., taken internally within a control logic implemented by the processor subsystem. This way, one or more autonomous operations of the surgical robotic system may be adjusted based on the presence and/or absence of the feature of interest. For example, the feedback action may one or more operational parameters of the surgical instrument or of a further surgical instrument, for example an aspiration rate of a micro vacuum pick, and/or its position and/or its orientation, e.g., its axial orientation.

Optionally, the processor subsystem is configured to effect the feedback action periodically or continuously for as long as the attribute meets the predetermined attribute criterion. A human operator may be preoccupied with controlling the surgical instrument and/or the procedural steps. Frequent and isolated feedback actions may distract the human operator. Such feedback actions may be frequent and isolated if at each position of the surgical instrument the feedback action is separately triggered. It has been found that less-intrusive feedback may be provided by providing the feedback action periodically, e.g., at regular time intervals, or continuously, for as long as the attribute meets the predetermined attribute criterion. As such, the feedback may be in principle independent on the position as long as the attribute does not change. Accordingly, as long as the feature of interest is detected, or remains absent, a relatively constant form of feedback may be provided. For example, a continuous audio signal may be provided in response to the attribute meeting the predetermined attribute criterion, and only if the attribute changes in a way that it does not meet the predetermined attribute criterion, the audio signal may be stopped. This may allow a human operator to focus more on the changes in attribute value without being repeatedly alerted to a same attribute value through isolated feedback actions.

Optionally, the processor subsystem is configured to determine the attribute of the feature of interest in the reflectivity depth profile further based on one or more previous reflectivity depth profiles obtained at one or more previous positions of the surgical instrument. The reliability of the feature detection may be improved by taking not only the current reflectivity depth profile into account, but also those obtained at one or more previous positions of the surgical instrument. For example, the previous positions may be near the current position and thereby the sensor data obtained at said previous positions may be further indicative of the attribute value at a current position. For example, if the feature of interest is found to be present at surrounding positions, this may increase the expectancy of the feature of interest being present at the current position, which in turn may be used to influence or steer the feature detection.

Optionally, the processor subsystem is configured to analyse the reflectivity depth profile using template matching or a machine learning-based technique.

Optionally, the feature of interest is an intraocular feature, for example one or a combination of:
- a bodily fluid in the vitreous cavity, for example blood,
- a floating object in the vitreous cavity, such as a vitreous floater or a partially detached retina,
- a foreign fluid in the vitreous cavity, such as a dye or silicone oil,
- a retinal membrane, such as epiretinal membrane or an internal limiting membrane,
- a retinal layer, and
- a subretinal structure, such as a blood vessel or tissue defect.

Optionally, the feature of interest is a tissue type, such as tumorous tissue.

Optionally, the processor subsystem is configured to build-up a map which is indicative of the attribute of the feature of interest by:
- controlling the actuator subsystem to move the surgical instrument along a trajectory;
- acquiring sensor data via the OCT probe at respective positions along the trajectory;
- analysing the sensor data to obtain a plurality of values of the attribute of the feature of interest along the trajectory;
- generating the map based on the plurality of values of the attribute and the respective positions; and
- outputting the map for use in or after the surgical procedure.

The surgical instrument may for example be moved lateral and/or longitudinal (e.g., axially). By assembling individual detection results at different positions into a map, such as a one-, two-, or three-dimensional map, a spatial overview may be obtained of the attribute value of the feature of interest in a region. For example, a map may be generated which spatially extents in lateral and/or longitudinal direction. Such spatial overviews may help in the interoperative procedure, e.g., to plan trajectories, further procedural steps, etc. In a specific example, the map may be a three-dimensional map covering a volume within the eye.

Optionally, the surgical robotic system comprises a user input interface for receiving positioning instructions from a user, wherein the processor subsystem is configured to control the actuator subsystem based on the positioning instructions to enable the user to determine the trajectory of the surgical instrument. The map may be built-up while the human operator is controlling the position of the surgical instrument. This way, the map may be generated automatically during the intraoperative procedure as a result of movement of the surgical instrument. Such functionality may also allow the user to purposefully control the position of the surgical instrument to expand the map into a previously uncovered direction. Advantageously, the above measures establish an unobtrusive mechanism to generate a map, without requiring a separate map generating phase which may otherwise interfere with the surgical procedure.

Optionally, the processor subsystem is configured to generate feedback for the user on how to cover an area or volume within the interior of the patient, e.g., within the eye, with the trajectory. It may be desirable to limit the movement of the surgical instrument within the patient's interior, for example within the eye, as any type of movement may run the risk of causing damage, e.g., due to collisions, forces exerted on the entry point to the eye, etc. At the same time, it may be desirable to build-up a map of the feature of interest via the movement of the surgical instrument. To support the map generation using a minimal or at least relatively low amount of movement, the processor subsystem may determine a trajectory to efficiently cover an area or volume within the interior of the patient and provide the trajectory for navigational guidance to the human operator, e.g., in form of visual, auditive, or haptic feedback. Such trajectory planning may be known per se from more remote fields, e.g., from surveillance, but their underlying techniques may be used and modified for the above-identified purpose.

Optionally, the processor subsystem is configured to incrementally build-up the map during the surgical procedure by adding, updating, or deleting a respective part of the map in response to a change in position of the surgical instrument.

Optionally, a change in position of the surgical instrument may be brought about by a change in eye pose, for example due to an intended reorientation of the eye to increase the reach of the surgical robotic system. The surgical robotic system may comprise one or more sensors to determine a change in eye pose, such as a sensor providing pupil tracking or a sensor for tracking a pose of the surgical arm or a sensor for tracking a trocar used as entry point for the surgical instrument. In response to such a change in eye pose, the processor subsystem may determine the attribute of the feature of interest at the changed position of the surgical instrument, and if applicable, effect a feedback action and/or update the map.

Optionally, the processor subsystem is configured to use color-coding in the map to indicate a presence of the feature of interest, for example by using a colour associated with dyeing a retinal membrane, such as trypan blue, brilliant blue G (BBG), or indocyanine green (ICG), to indicate the presence of the retinal membrane. A human operator specializing in intraocular procedures may be well-familiar with such dyes and therefore the human operator may easily interpret the map.

Optionally, the processor subsystem is configured to analyse the map to determine a position for the surgical instrument at which to perform an operative action. Having generated a map of the feature of interest, an operative action may be planned based on the map. For example, the operative action may be a grasping action or a peeling action, and the processor subsystem may use the map to determine where, e.g., at which lateral position and from which direction, to best grasp and/or peel a feature of interest. In a specific example, a map of an epiretinal membrane may be used to determine where to best grasp and start peeling the epiretinal membrane.

Optionally, the surgical robotic system may be configured for microsurgery. The surgical instrument may thus be a microsurgical instrument.

Optionally, the surgical robotic system may be configured for minimally invasive surgery. The surgical instrument may thus be a surgical instrument for use in such minimally invasive surgery.

Optionally, the surgical robotic system may be configured for intraocular surgery. The surgical instrument may thus be an intraocular surgical instrument.

It will be appreciated by those skilled in the art that two or more of the above-mentioned embodiments, implementations, and/or aspects of the invention may be combined in any way deemed useful.

Modifications and variations of the computer-implemented method and/or the computer-readable medium, which correspond to the described modifications, variations, and optional aspects of the surgical robotic system, may be carried out by a person skilled in the art on the basis of the present description.

### BRIEF DESCRIPTION OF THE DRAWINGS

These and other aspects of the invention are apparent from and will be elucidated with reference to the embodiments described hereinafter. In the drawings,
Fig. 1 shows a schematic representation of a surgical robotic system;
Fig. 2 shows a surgical instrument passing through a trocar during minimally invasive surgery, the surgical instrument having four degrees of freedom (DoFs);
Fig. 3 shows a joint diagram illustrating the kinematics of a movable arm part of a surgical arm for use in minimally invasive surgery;
Fig. 4 shows a joint diagram illustrating the kinematics of a motion controller;
Fig. 5 shows a surgical instrument in an eye according to an embodiment of the invention;
Figs. 6A and 6B show a detection of an attribute of a feature of interest;
Fig. 7 shows a map showing a presence of a feature of interest;
Fig. 8 schematically shows a method for controlling a surgical robotic system during a surgical procedure; and
Fig. 9 shows a non-transitory computer-readable medium;

It should be noted that items which have the same reference numbers in different figures, have the same structural features and the same functions, or are the same signals. Where the function and/or structure of such an item has been explained, there is no necessity for repeated explanation thereof in the detailed description.

### List of reference numerals

The following list of references and abbreviations is provided for facilitating the interpretation of the drawings and shall not be construed as limiting the claims.
20 user interface subsystem
22 user inputs
30 sensor
32 sensor data
40 processor subsystem
42 actuation commands
60 actuator subsystem
62 actuation of surgical arm
80 surgical arm
82 movable arm part
100 surgical robotic system
104 *e̅ₓ*, axis of coordinate system fixed to the instrument tip, orthogonal to the instrument longitudinal axis
105 *e̅_{y},* axis of coordinate system fixed to the instrument tip, orthogonal to the instrument longitudinal axis
106 *e̅_{z}*, axis of a cartesian coordinate system, aligned with the instrument longitudinal axis
107 *ϕ*, rotation of surgical instrument, laterally displacing its tip
108 *ψ*, rotation of surgical instrument, laterally displacing its tip
109 *z*, longitudinal (along its longitudinal axis) translation of surgical instrument, or penetration direction, or advancing direction
110 θ, rotation of surgical instrument around its longitudinal axis
111 *φ*, rotational DoF of a movable arm part
112 *ψ*, rotational DoF of a movable arm part
113 *z*, translational DoF of a movable arm part
114 *θ*, rotational DoF of a movable arm part
115 *φₘ,* rotational DoF of motion controller
116 *ψₘ*, rotational DoF of motion controller
117 *zₘ*, translational DoF of motion controller
118 *θₘ*, rotational DoF of motion controller
119 surgical instrument
121 sensor or sensor part
122 surgical instrument tip
123 surgical target
124 trocar
125 remote centre of motion (RCM)
126 button on motion controller gripper
127 optical beam
200 eye
210 retina
220 first layer of tissue
230 second layer of tissue
240 feature of interest
250 audio signal
252 silenced audio signal
300 map
310 presence of feature of interest
400 method of controlling a surgical robotic system
410 controlling actuator subsystem
420 monitoring for attribute of feature of interest
430 acquiring sensor data
440 analysing reflectivity depth profile
450 determining of attribute value matches predetermined criterion
460 effecting feedback action
500 non-transitory computer readable medium
510 data representing computer program

### DESCRIPTION OF EMBODIMENTS

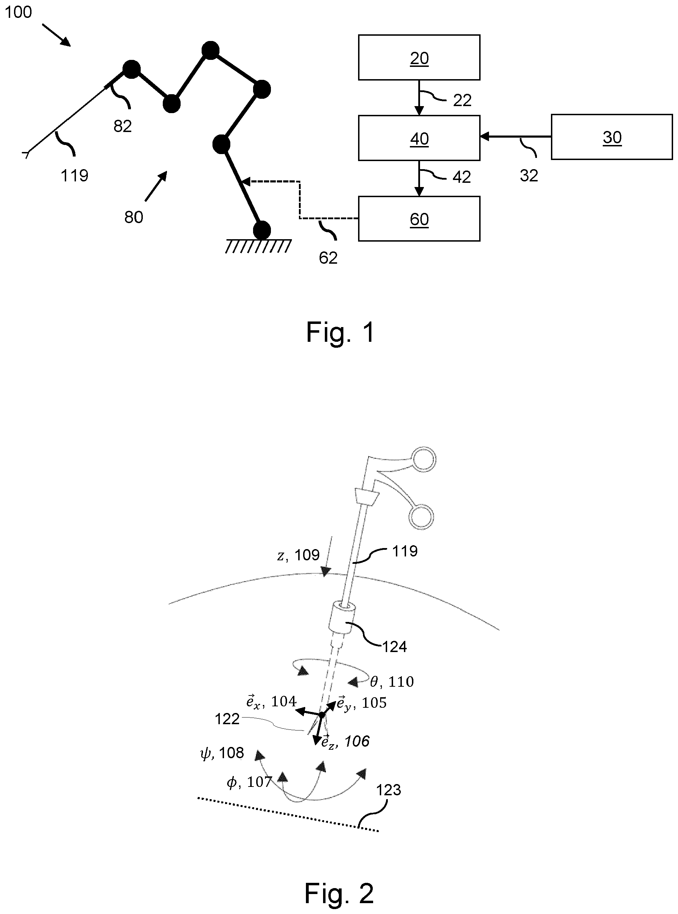

The following embodiments relate to a surgical robotic system for use in a surgical procedure. During the surgical procedure, the surgical robotic system may monitor for an attribute of a feature of interest using an OCT probe which is attached to or integrated into an insertable part of the surgical instrument for being inserted into an interior of a patient. The following describes such monitoring within the context of a specific surgical robotic system, namely a surgical robotic system which is configured for intraocular surgery and described with reference to, inter alia, Figs. 1-4. Nonetheless, the embodiments described in this specification are not confined solely to this type of surgical robotic system, nor to the field of intraocular surgery. Rather, the embodiments can be adapted and applied to other types of surgical robotic systems for use in intraocular procedures, as well as to other types of surgical robotic systems for use in other types of surgical procedures, such as microsurgical procedures, minimally invasive procedures, endoluminal procedures, etc. Any references to intraocular surgery and intraocular structures are therefore to be understood as applying also to other types of surgical procedures and corresponding types of anatomical structures.

**Fig. 1** schematically shows a surgical robotic system 100 for use in an intraocular surgical procedure. The surgical robotic system 100 may comprise a surgical arm 80. The surgical arm 80 may comprise a movable arm part 82 which may comprise an end effector (not explicitly shown in Fig. 1) for holding a surgical instrument 119. For example, the surgical instrument 119 may be an irrigation/aspiration instrument for irrigating or aspirating fluid at a desired target location, a photocoagulation instrument for applying optical energy at a desired distance from a target, a vitrectomy instrument for cutting and aspirating the vitreous humor or other fluidics at a desired distance from a target, a tissue manipulating instrument, which may need to be placed at the interface of a tissue, or the like. To hold the surgical instrument 119, the end effector may comprise an instrument connector to which the surgical instrument 119 may be mounted. The movable arm part 82 may have at least three degrees-of-freedom (DoF) to enable longitudinal movement of the surgical instrument towards a surgical target within the eye and lateral movement of the surgical instrument in a plane normal to said longitudinal movement. Here, longitudinal movement may refer to a movement of the surgical instrument 119 along its longitudinal axis (not separately shown in Fig. 1). In some embodiment, the surgical robotic system 100 may be configured to enable lateral movement in a curved plane. The curvature of the curved plane may for example follow a curvature of the interior of the eye. Such type of lateral movement along a curved plane may be obtained by the surgical robotic system adjusting the longitudinal position of the surgical instrument 119 during lateral movement. The shape of the curved plane may for example be determined based on a model of the eye or an intraocular structure, e.g., the retina.

In some embodiments, the surgical robotic system 100 may further comprise a user interface subsystem 20 for receiving user inputs 22 from a user. The user interface subsystem 20 may thus represent at least a user *input* interface, and in some embodiments, as also discussed elsewhere, additionally or alternatively a user *output* interface. The user inputs 22 may for example comprise positioning instructions from a human operator, e.g., a surgeon, to enable the human operator to determine a trajectory of the surgical instrument. In some embodiments, the positioning instructions may be positioning commands for directly controlling the movement of the surgical instrument. In other embodiments, the positioning instructions may be provided as input during a planning procedure in which the trajectory of the surgical instrument is planned. Other types of user inputs may for example include confirmation inputs indicating instructions to continue with a procedure or procedural step, and/or input information, such as indicating an operating parameter and/or an indication of a surgical target position or the like. Examples of user input interfaces for receiving user inputs from a user include, but are not limited to, a keyboard, a mouse, a touch-sensitive surface, a joystick, a foot pedal, a microphone, a gesture recognition system, etc. The user input interface may employ any suitable input modality, such as touch, push-actions, voice commands, eye movements, gesture recognition, etc.

The surgical robotic system 100 may further comprise an actuator subsystem 60 configured and arranged for actuating the movable arm part to effect the longitudinal movement and lateral movement of the surgical instrument. The actuator subsystem 60 may comprise any suitable actuator(s), e.g., from the field of surgical robots, or from the more general field of actuators. In particular, the actuator subsystem 60 may comprise a plurality of actuators which together provide the actuation of the movable arm part 60 along the three or more DoFs. Accordingly, it will be appreciated that any reference to a specific configuration of the actuator subsystem 60 may be understood as referring to a (joint) configuration of such a plurality of actuators.

Fig. 1 shows the actuation of surgical arm 80 schematically, namely as a dashed line 62. It is noted that, although shown separately of the surgical arm 80, the actuator subsystem 60 may be integrated into, or mounted to, the surgical arm 80.

The surgical robotic system 100 may further comprise a sensor 30. The sensor 30 may be or comprise an optical coherence tomography (OCT) probe. The OCT probe may for example be an optical fibre which is attached to or integrated in the surgical instrument 119, with the optical fibre being connected to a remotely located OCT sensor. In other examples, the OCT probe may comprise an OCT sensor which is directly attached to or directly integrated in the surgical instrument 119. The sensor data 32 provided by the OCT probe may comprise A-scans, which may comprise line measurements and defined as an intensity as a function of the distance, B-scans forming a 2D image, C-scans, e.g., from multiple B-scans, or the like. It is noted that even though the sensor 30 is shown in Fig. 1 to be separate from the surgical instrument 119, the sensor or part of the sensor may be attached to or integrated in the surgical instrument 119. In other examples, the sensor 30 may, in addition or alternatively to the OCT probe, comprise a stereo camera arranged for image capture through a microscope, an optical interferometric sensor integrated in or attached to the surgical instrument 119, a time-of-flight sensor integrated in or attached to the surgical instrument and an ultrasonic sensor integrated in or attached to the surgical instrument etc. In some embodiments, the surgical robotic system 100 may comprise a microscope which may be communicatively coupled to the surgical robotic system 100.

The surgical robotic system 100 may further comprise a processor subsystem 40 configured for controlling the actuator subsystem 60 to control a position of the surgical instrument 119 during the intraocular procedure. For the purpose of controlling the actuator subsystem 60, the processor subsystem 40 may provide actuation commands 42 to the actuator subsystem. For example, through such control, the surgical instrument 119 may be positioned relative to an intraocular surface, which may be a surface which is of relevance to the surgical procedure. In a specific example, in an epiretinal membrane peeling procedure, the surgical instrument 119 may be positioned relative to the surface of the retina to grasp and peel the epiretinal membrane starting from an edge of the epiretinal membrane. Another example is that during cataract surgery, the surgical instrument 119 may be positioned relative to the surface of the lens or the capsular bag. Yet another example is that during glaucoma surgery, the surgical instrument 119 may be positioned relatively to the surface of the trabecular meshwork. The following continues to refer to the surgical instrument 119 being positioned laterally with respect to an intraocular surface. It will be appreciated, however, that the surgical instrument may also be moved in any other manner, e.g., relative to another reference object, or moved non-laterally, for example longitudinally. Any examples or embodiments referring to the lateral positioning relative to an intraocular surface may thus equally apply to any other positioning of the surgical instrument, for example the aforementioned longitudinal positioning.

As also discussed elsewhere, the processor subsystem 40 may function in an operator control mode in which the processor subsystem 40 directly carries out positioning commands received from a human operator, or in an autonomous control mode in which the processor subsystem 40 autonomously controls the position of the surgical instrument, e.g., according to a pre-planned trajectory and sensor data, or in a semi-autonomous control mode which combines aspects of the operator control mode and the autonomous control mode. During the intraocular procedure, and thus during, or time-alternatingly with, the control of the actuator subsystem 60, the processor subsystem 40 may monitor for an attribute of a feature of interest on or below the intraocular surface by repeatedly, at respective positions of the surgical instrument, acquiring sensor data 32 via the OCT probe. The sensor data may be indicative of a reflectivity depth profile originating from structures within the radiating beam of the OCT probe. The processor subsystem 40 may analyse the reflectivity depth profile to determine an attribute of the feature of interest in the reflectivity depth profile, for example using template matching or a machine-learning based technique. The processor subsystem 40 may determine if the attribute meets a predetermined attribute criterion, for example whether an attribute in form of a presence status is equal 'present', and if so, effect a feedback action. As such, determining the attribute may include identifying whether the feature of interest is present in the depth profile, with the attribute being in this example a presence or presence status of the feature of interest.

The feedback action may for example comprise adjusting the control of the actuator subsystem, for example to slow down or stop a movement of the surgical instrument. Another example of a feedback action may be the control of a further surgical instrument held by the surgical robotic system. Yet another example of a feedback action may be the generation and outputting of a sensory perceptible feedback signal to a user. The sensory perceptible feedback signal may for example be an audible signal, a haptic signal, and/or a visual signal. For example, the processor subsystem 40 may be configured to output the sensory perceptible feedback signal via the user interface subsystem 20. In such an example, the user interface subsystem 20 may thus at least represent an output interface. For example, the sensory perceptible feedback signal may be rendered visually, e.g., as an overlay over an image of the surgical field, as an audio output, e.g., via a speaker of the user interface subsystem 20, and/or as a visual numerical output, e.g., on a display of the user interface subsystem 20. Another example is that a haptic signal may be provided via the user input interface, e.g., by providing a haptic signal via a motion controller. In some examples, the sensory perceptible feedback signal may be provided periodically or continuously for as long as the attribute meets the predetermined attribute criterion. For example, the sensory perceptible feedback signal may be provided as long as the feature of interest is detected to be beneath the tip of the surgical instrument.

**Fig. 2** shows a surgical instrument 119 passing through a trocar 124 during minimally invasive surgery. For example, in case of vitreoretinal surgery, the trocar 124 may be placed in the sclera. Rotating around and translating through the trocar may be possible in four DoF, e.g., the rotations *ϕ* 107, *ψ* 108, *θ* 110 and the translation z 109 to approach or penetrate a surgical target 123. Further shown are a tip 122 of the surgical instrument 119 and three axes 104-106 of a coordinate system fixed to the instrument tip 122, with *e̅_{z}* 106 aligned with the longitudinal axis of the surgical instrument 119. Rotations *ϕ* 107 and *ψ* 108 may result in a lateral displacement of the instrument tip 122, respectively in the direction *e̅_{y}* 105 and in the direction *e̅ₓ* 104. The translation z 109 may result in a longitudinal movement of the surgical instrument tip 122.

The surgical robotic system 100 may be used in an intraocular surgical procedure, such as a minimally invasive surgical procedure as described above.

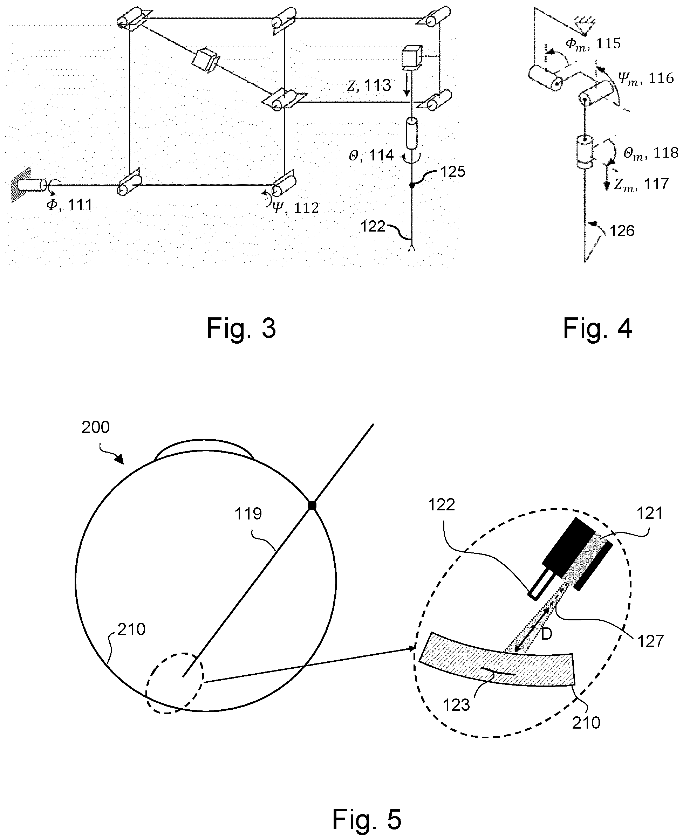

**Fig. 3** shows a joint diagram illustrating the kinematics of a movable arm part of a surgical arm for use in an intraocular surgical procedure, and in particular a minimally invasive surgery. In the example of Fig. 3, the surgical robotic system comprises a surgical arm, with the surgical arm comprising a movable arm part having DoFs *Φ* 111, *Ψ* 112, Z 113 and *Θ* 114, allowing instrument motions 107-110 as previously shown in Fig. 2, resulting in movements of the surgical instrument tip 122. The DoFs may be arranged such that there is a point on the surgical instrument 122 that does not move in space, termed the remote centre of motion (RCM) 125. By moving the base of the surgical arm, the movable arm part may be positioned such that the remote centre of motion 125 of the surgical instrument is positioned at the trocar. Respective actuators may be arranged to effect movement in all four DoFs 111-114.

As previously discussed with reference to Fig. 1, the surgical robotic system may comprise a user input interface for receiving user inputs, such as positioning instructions, from a human operator, e.g., a surgeon or healthcare provider. In some embodiments, the user interface may comprise or be constituted by a motion controller such as a joystick. In some embodiments, the motion controller may be an external device with which the user interface or the processor subsystem 40 is configured to interface. In some embodiments, the motion controller may be comprised in the surgical robotic system as a further component, e.g., configured to interface with the processor subsystem 40 and/or the user interface subsystem 20. **Fig. 4** shows a joint diagram illustrating the kinematics of such a motion controller. Here, the motion controller is shown to have DoFs *Φₘ* 115, *Ψₘ* 116, *Zₘ* 117 and *Θₘ* 118. The user may provide user inputs, such as positioning commands to directly control the movement of the movable arm part, for example by holding the motion controller at a gripper part, pressing the button 126 and moving the gripper part of the motion controller in space.

**Fig. 5** shows a surgical instrument 119 in an eye 200. The tip 122 of the surgical instrument 119 is also shown magnified. The tip 122 of the surgical instrument 119 may be an operative tip in that the tip may be operatively involved in the intraocular procedure. As illustrated on the left hand side of Fig. 5, the surgical instrument 119 is shown inserted into the eye 200. The surgical instrument 119 may be approaching the retina 210 as illustrated in this figure, although this is merely exemplary and the invention is not limited thereto. In some embodiments, the surgical instrument 119 may approach another area, structure, or tissue layer of the eye, such as a capsular bag or a part of the eye's drainage system. The tip 122 of the surgical instrument 119 is illustrated on the right hand side of Fig. 5, magnified. In this example, a surgical target 123 is located in the retina. The surgical instrument 119 may for example be an irrigation/aspiration instrument for irrigating or aspirating fluid at a desired target location, a photocoagulation instrument for applying optical energy at a desired distance from a target, a vitrectomy instrument for cutting and aspirating the vitreous humor or other fluidics at a desired distance from a target, a tissue manipulating instrument, which may need to be placed more or less exactly at the first interface of a tissue, or the like. The tip 122 of the surgical instrument 119 may for example be an injection lumen. A sensor or sensor component 121, such as an optical fibre, may be integrated in or attached to the surgical instrument 119. In some embodiments, the sensor 30 may comprise an optical fibre (as shown) coupled to an OCT sensor and configured to emit an optical beam 127 to capture cross-sectional images of the retina 210 and other structures within the eye. The optical fibre 121 may be recessed from the tip 122 of the surgical instrument 119, which may allow capturing sensor data whilst the tip 122 of the surgical instrument 119 may be piercing or penetrating a tissue layer.

Using the OCT probe, the surgical robotic system may obtain sensor data which is indicative of a reflectivity depth profile originating from structures on or below the intraocular surface. As also discussed elsewhere, the processor subsystem may analyse the reflectivity depth profile to determine an attribute of a feature of interest in the reflectivity depth profile. The feature of interest may for example be a retinal membrane, such as epiretinal membrane or an internal limiting membrane, a retinal layer, a subretinal structure, such as a blood vessel or tissue defect (e.g., a pathology such as a detached retina or subretinal fluid, or a retinal hole, a gap between layers or a detached membrane). Since the OCT probe may be longitudinally forward facing, the sensor data may be indicative of a presence of the feature of interest laterally forward of the tip of the surgical instrument, which may be considered to be 'beneath' the tip of the surgical instrument. It is noted that when the surgical instrument is operated in the periphery of the eye, or in the vicinity of the posterior side of the lens in vitrectomy, a feature of interest may be positioned more sideward than longitudinally forward. To account for such sideward positioning, the OCT probe may be a multi-directional probe with axial and side-viewing capability so as to be able to detect the feature of interest also sideward to the tip of the surgical instrument.

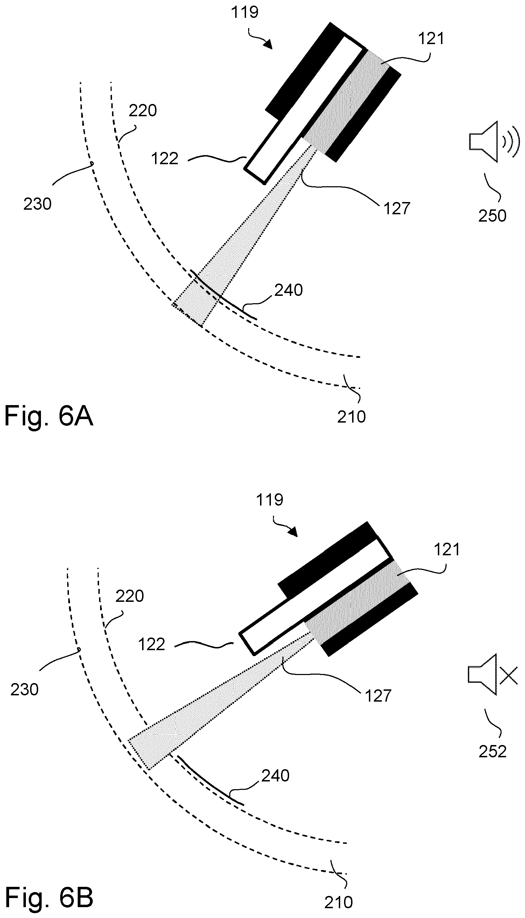

**Fig. 6A** shows a surgical instrument 119 at a given first lateral position relative to the retina 210 (which is shown in Figs. 6A and 6B to comprise retinal layers 220, 230). Whilst at the first lateral position, the attribute of a feature of interest may be determined, for example using the OCT probe coupled to, attached to, or integrated in the surgical instrument 119, as shown in Fig. 5. Preferably, the sensor measurement is performed whilst the surgical instrument 119 is stationary. The feature of interest may in this example be a retinal membrane, in particular the epiretinal membrane 240, which may have formed over a part of the surface of the retina 210. By way of the aforementioned analysis of the reflectivity depth profile, the processor subsystem may determine whether the epiretinal membrane 240 is present at or near the current lateral position of the surgical instrument. Fig. 6A shows this to be the case, i.e., the attribute may be 'present'. The processor subsystem may then take a feedback action, such as the sounding of an audio signal 250 (shown schematically in Figs. 6A and 6B using a loudspeaker symbol). **Fig. 6B** illustrates the surgical instrument 119 having moved laterally to a second position in the eye 200 at which the epiretinal membrane 240 is not present anymore. The lateral movement of the tip 122 of the surgical instrument 122 may be obtained by a rotation about the RCM (not shown in Fig. 6B) of the surgical instrument. As the epiretinal membrane 240 is not present anymore at or near the second lateral position, the processor subsystem may not take a feedback action, and if the feedback action is otherwise performed continuously or periodically in response to the attribute of the feature of interest, stop performing the feedback action. Fig. 6B illustrates the latter by showing that the audio signal 252 is silenced.

It will be appreciated that while Figs. 6A and 6B show the feedback action being taken in response to the feature of interest being present, alternatively the feedback action may be taken in response to the feature of interest being absent.

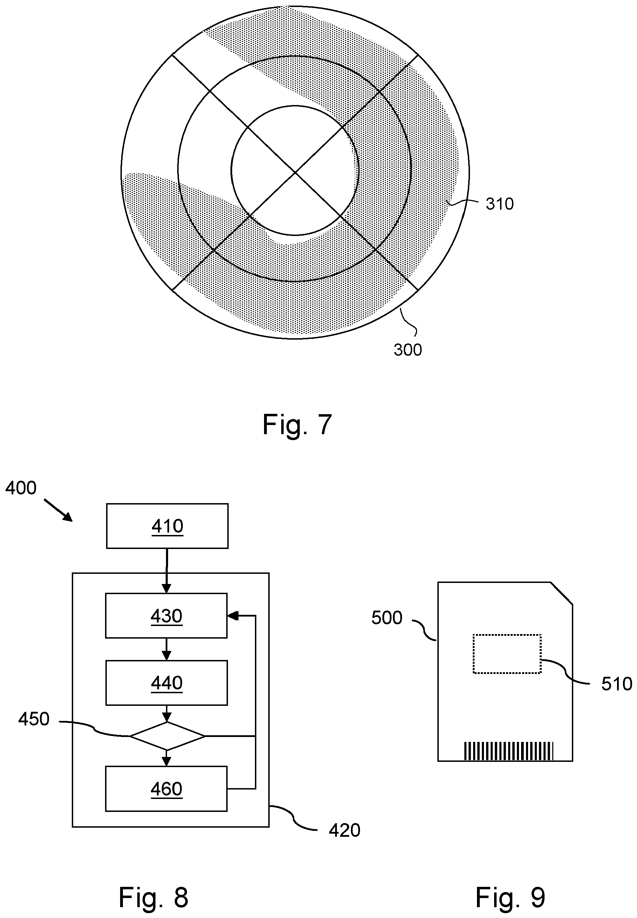

**Fig. 7** shows a map 300 showing an attribute of a feature of interest, being in this specific example a presence status in form of a binary representation (e.g., yes, no) of the presence 310 of a feature of interest. Such a map 300 may be built-up by the processor subsystem by, at respective lateral positions, determining the attribute of the feature of interest so as to obtain a plurality of values of the attribute of the feature of interest, and generating the map based on the plurality of values of the attribute and the respective lateral positions. For example, the different lateral positions may lie along a trajectory along which the surgical instrument is moved, either autonomously, semi-autonomously or under direct control of a human operator. The map 300 may be output by a map generating function and may be used internally by the processor subsystem, for example in further decision making by the processor subsystem, and/or externally, for example by the map being displayed or otherwise rendered to be perceived by a human operator. As noted elsewhere in this specification, the map may additionally or alternatively extent in the longitudinal direction based on longitudinal movement of the surgical instrument. The map may thus be a map having at least one longitudinal dimension, which may also be referred to as 'depth' or 'axial' dimension. A specific example of map having at least one longitudinal dimension is a map which covers a volume within the vitreous cavity, for example indicating the presence and/or one or more characteristics of bodily fluids, foreign fluids, and/or floating objects.

In some embodiments, the processor subsystem may incrementally build-up the map 300 during operation of the surgical robotic system, for example while the human operator steers the surgical instrument along a particular trajectory, or when (semi-)autonomously carrying out a trajectory. In some embodiments, the processor subsystem may determine how to cover an intraocular area or volume optimally or at least adequately with a trajectory and may provide feedback to a human operator which is indicative of the trajectory. The map may be updated during operation of the surgical robotic system. For example, when the surgical instrument is positioned at new position, a respective part of the map may be added to reflect the attribute value of the feature of interest at the new position. Another example is that when the surgical instrument is positioned again at a previously visited position, the respective part of the map may be updated to reflect the latest attribute value of the feature of interest. Yet another example is that a part of the map may be deleted when new sensor data invalidates a previous determination of the attribute of the feature of interest. For example, if the feature of interest is determined to be absent at a current position, it may be concluded that a previous determination that the feature of interest is present at a previous position was erroneous. Accordingly, the part of the map pertaining to the previous position may be deleted or its attribute value may be corrected.

While various types of visualizations of the map are conceivable, in some embodiments, the processor subsystem may be configured to specifically use color-coding of the map 300 to indicate a presence of the feature of interest, for example by using a colour associated with dyeing a retinal membrane, such as trypan blue, brilliant blue G (BBG), or indocyanine green (ICG), to indicate the presence of the retinal membrane. In some embodiments, the processor subsystem may be configured to analyse the map 300 to determine a position for the surgical instrument at which to perform an operative action, such as a grasping action or a peeling action which may be performed in relation to the feature of interest. For example, if the feature of interest is an epiretinal membrane, the processor subsystem may analyse the map 300 to determine a lateral position at which an edge of the epiretinal membrane can be grasped by the surgical instrument to peel or further peel the epiretinal membrane.

An example of the use of the surgical robotic system outside of intraocular surgery may be one in which the surgical robotic system is configured for selective tumour removal, for example in trans-oral laser microsurgery in the vocal cord region of the throat. In such examples, the surgical robotic system may be provided with a surgical instrument which combines an OCT probe with an optical system for delivering laser radiation to the tissue. The sensor data from the OCT probe may be used to differentiate healthy from tumorous tissue. Additionally, in the sensor data, critical structures such as vessels or nerves may be identified. Based on the presence of the features of interest `tumorous tissue' and 'no critical structure (e.g. nerve, vessel)', a feedback action may be triggered which activates the laser or prevents activation of the laser at lateral positions. Additionally, or alternatively, the processor subsystem may be configured to generate a map of tissue regions to be treated based on a scan of the relevant tissue region. This scan may be obtained by laterally scanning the tip of the OCT probe in two directions while recording axial OCT distance scans (OCT-A scans). Based on the generated map of regions to be treated, an automated treatment plan may be generated which may be executed for an automated tumour removal.

Yet another example is that the surgical robotic system may use fluorescence imaging to differentiate between scar tissue or recurrent cancer beyond the line of sight of Optical Precision Microscope Instrumentation (OPMI). For that purpose, the surgical robotic system may detect features of interest such as tortuous and dilated new blood vessels in the area where cancer was previously removed.

In general, the processor subsystem described in this specification may comprise one or more (micro)processors which execute appropriate software. The software implementing the functionality of the processor subsystem may have been downloaded and/or stored in a corresponding memory or memories, e.g., in volatile memory such as RAM or in non-volatile memory such as Flash. Alternatively, the processor subsystem may be implemented in the form of programmable logic, e.g., as a Field-Programmable Gate Array (FPGA). Any input and/or output interfaces may be implemented by respective hardware and/or software interfaces. In general, the processor subsystem, input interfaces, and/or output interfaces may each be implemented in the form of a circuit or circuitry. The processor subsystem may also be implemented in a distributed manner, e.g., involving different devices or apparatus.

**Fig. 8** shows a method 400 of controlling a surgical robotic system during a surgical procedure. The method 400 may comprise controlling 410 an actuator subsystem to control a position of the surgical instrument during a surgical procedure, and simultaneously or alternatingly in time with said controlling, monitoring 420 for an attribute of a feature of interest by repeatedly, at respective positions of the surgical instrument, acquiring 430 sensor data via the OCT probe, analysing 440 the reflectivity depth profile to determine an attribute of the feature of interest in the reflectivity depth profile, determining 450 if the attribute meets a predetermined attribute criterion, and if so, effecting 460 a feedback action.

It is noted that any of the methods described in this specification, for example in any of the claims, may be implemented on a computer as a computer implemented method, as dedicated hardware, or as a combination of both. Instructions for the computer, e.g., executable code, may be stored on a computer-readable medium 500 as for example shown in Fig. 9, e.g., in the form of a series 510 of machine-readable physical marks and/or as a series of elements having different electrical, e.g., magnetic, or optical properties or values. The executable code may be stored in a transitory or non-transitory manner. Examples of computer-readable mediums include memory devices, optical storage devices, integrated circuits, servers, online software, etc. Fig. 9 shows by way of example a memory card 500.

It should be noted that the above-mentioned embodiments illustrate rather than limit the invention, and that those skilled in the art will be able to design many alternative embodiments without departing from the scope of the appended claims.

In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. Expressions such as "at least one of" when preceding a list or group of elements represent a selection of all or of any subset of elements from the list or group. For example, the expression, "at least one of A, B, and C" should be understood as including only A, only B, only C, both A and B, both A and C, both B and C, or all of A, B, and C. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A surgical robotic system (100) for use in an intraocular procedure, comprising:
- a surgical arm (80) comprising a movable arm part (82), the movable arm part comprising an end effector for holding a surgical instrument (119);
- an optical coherence tomography, CT, probe (30, 121), wherein the OCT probe (30, 121) is configured to attach to or integrate into an intraocular part of the surgical instrument (119) for being inserted into an interior of an eye;
- an actuator subsystem (60) configured to actuate the movable arm part (82);
- a processor subsystem (40) configured to control the actuator subsystem (60) to control a position of the surgical instrument (119) during the intraocular procedure, wherein the processor subsystem (40) is further configured to monitor for a feature of interest (240) by repeatedly, at respective positions of the surgical instrument (119):
- acquire sensor data (32) via the OCT probe (30, 121), wherein the sensor data (32) is indicative of a reflectivity depth profile originating from structures within a beam of the OCT probe;
- analyse the reflectivity depth profile to determine an attribute of the feature of interest (240) in the reflectivity depth profile; and
- if the attribute meets a predetermined attribute criterion, effect a feedback action (250).

2. The surgical robotic system (100) according to claim 1, wherein the processor subsystem (40) is configured to effect the feedback action by:
- generating and outputting a sensory perceptible feedback signal, such as an audible signal (250), a haptic signal, and/or a visual signal;
- adjusting the control of the actuator subsystem (60), for example to slow down or stop a movement of the surgical instrument (119); and/or
- control the surgical instrument (119) or a further surgical instrument held by the surgical robotic system.

3. The surgical robotic system (100) according to claim 1 or 2, wherein the processor subsystem (40) is configured to effect the feedback action (250) periodically or continuously for as long as the attribute meets the predetermined attribute criterion.

4. The surgical robotic system (100) according to any one of claims 1 to 3, wherein the processor subsystem (40) is configured to determine the attribute of the feature of interest (240) in the reflectivity depth profile further based on one or more previous reflectivity depth profiles obtained at one or more previous positions of the surgical instrument (119).

5. The surgical robotic system (100) according to any one of claims 1 to 4, wherein the processor subsystem (40) is configured to analyse the reflectivity depth profile using template matching or a machine learning-based technique.

6. The surgical robotic system (100) according to any one of claims 1 to 5, wherein the feature of interest (240) is an intraocular feature of interest, for example one or a combination of:
- a bodily fluid in the vitreous cavity, for example due to a haemorrhage,
- a floating object in the vitreous cavity, such as a vitreous floater or a partially detached retina,
- a foreign fluid in the vitreous cavity, such as a dye or silicone oil,
- a retinal membrane, such as epiretinal membrane or an internal limiting membrane,
- a retinal layer, and
- a subretinal structure, such as a blood vessel or tissue defect.

7. The surgical robotic system (100) according to any one of claims 1 to 6, wherein the processor subsystem (40) is configured to build-up a map which is indicative of the attribute of the feature of interest by:
- controlling the actuator subsystem (60) to move the surgical instrument (119) along a trajectory;
- acquiring sensor data (32) via the OCT probe (30, 121) at respective positions along the trajectory;
- analysing the sensor data (32) to obtain a plurality of values of the attribute of the feature of interest along the trajectory;
- generating the map (300) based on the plurality of values of the attribute and the respective positions; and
- outputting the map for use in or after the intraocular procedure.

8. The surgical robotic system (100) according to claim 7, further comprising a user input interface (20) for receiving positioning instructions from a user, wherein the processor subsystem (40) is configured to control the actuator subsystem (60) based on the positioning instructions to enable the user to determine the trajectory of the surgical instrument (119).

9. The surgical robotic system (100) according to claim 8, wherein the processor subsystem (40) is configured to generate feedback for the user on how to cover an area or volume within the interior of the eye with the trajectory.

10. The surgical robotic system (100) according to any one of claims 7 to 9, wherein the processor subsystem (40) is configured to incrementally build-up the map during the intraocular procedure by adding, updating, or deleting a respective part of the map (300) in response to a change in position of the surgical instrument (119).

11. The surgical robotic system (100) according to any one of claims 7 to 10, wherein the processor subsystem (40) is configured to use color-coding in the map (300) to indicate a presence of the feature of interest (240), for example by using a colour associated with dyeing a retinal membrane, such as trypan blue, brilliant blue G (BBG), or indocyanine green (ICG), to indicate the presence of the retinal membrane.

12. The surgical robotic system (100) according to any one of claims 7 to 11, wherein the processor subsystem (40) is configured to analyse the map (300) to determine a position for the surgical instrument (119) at which to perform an operative action.

13. The surgical robotic system (100) according to claim 12, wherein the operative action is a grasping action or a peeling action.

14. A computer-implemented method (400) for controlling a surgical robotic system during an intraocular procedure, wherein the surgical robotic system comprises:
- a surgical arm comprising a movable arm part, the movable arm part comprising an end effector for holding a surgical instrument;
- an optical coherence tomography, OCT, probe, wherein the OCT probe is configured to attach to or integrate into an intraocular part of the surgical instrument for being inserted into an interior of an eye;
- an actuator subsystem configured to actuate the movable arm part;
wherein the method comprises:
- controlling (410) the actuator subsystem to control a position of the surgical instrument during the intraocular procedure; and
- simultaneously or alternatingly in time with said controlling, monitoring (420) for a presence of a feature of interest by repeatedly, at respective positions of the surgical instrument:
- acquiring (430) sensor data via the OCT probe, wherein the sensor data is indicative of a reflectivity depth profile originating from structures within a beam of the OCT probe;
- analysing (440) the reflectivity depth profile to determine an attribute of the feature of interest in the reflectivity depth profile; and
- if the attribute meets a predetermined attribute criterion, effecting (460) a feedback action.

15. A transitory or non-transitory computer-readable medium (500) comprising data (510) representing a computer program, the computer program comprising instructions for causing a processor system to perform the method according to claim 14.
